# EUROPEAN PATENT SPECIFICATION

(11) **EP 4 464 289 B1**
(45) Date of publication and mention of the grant of the patent: **21.01.2026**
(21) Application number: 24176350.7
(22) Date of filing: 16.05.2024
(51) Int. Cl.: A61F 9/00

(54) **DROP-TYPE DRUG TUBE AND ITS MANUFACTURING METHOD**
TROPFENTUBE FÜR MEDIKAMENTE UND VERFAHREN ZU IHRER HERSTELLUNG
TUBE DE MÉDICAMENT DE TYPE GOUTTE ET SON PROCÉDÉ DE FABRICATION

(30) Priority: 18.05.2023 KR 20230064199
(43) Date of publication of application: 20.11.2024
(73) Proprietor: Wells Bio, Inc., Seoul 07795 (KR)
(72) Inventor: CHOI, Young-Ho, New Jersey (US); LEE, Min-Jun, 05118 Gwangjin-gu (KR)
(74) Representative: Gille Hrabal Partnerschaftsgesellschaft mbB Patentanwälte

(56) References cited:
- EP-A2- 1 075 875
- WO-A1-02/074374
- WO-A1-2007/045360
- US-A1- 2001 027 301
- US-A1- 2003 146 245

## Description

### BACKGROUND

The present disclosure relates to a drop-type drug tube method..

Recently, the burden on the eyes has increased due to an increase in various visual media such as computers, tablets, and TVs, an increase in mobile content, and deterioration in ambient air environment. In addition, as external beauty becomes more important, the wearing of lenses that directly stimulate the cornea is increasing rather than wearing glasses that do not directly irritate the cornea of the eye. Thus, use of eye drops such as artificial tears or eye water is increasing.

As the use of the eye drops such as the artificial tears and the eye water increases, research is being actively conducted on containers that are capable of storing the eye drops and allows users to easily use the eye drops. That is, research on eye drop tubes is actively conducted, and the scope of use of eye drop tubes is gradually increasing, as the eye drop tubes are used to store and use not only eye drops but also other reagents.

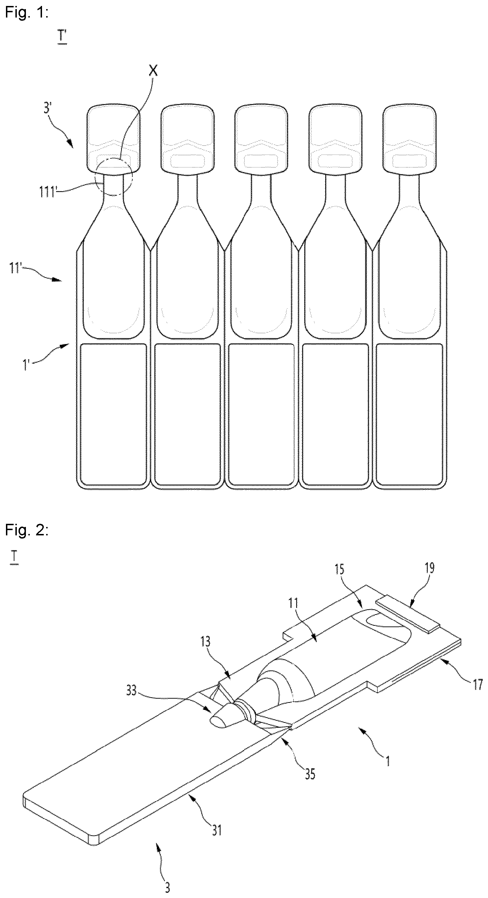

FIG. 1 is a view illustrating a drop-type drug tube according to a related art. Referring to FIG. 1, a drop-type drug tube T' according to the related art may define an outer appearance thereof and may include a body part 1' that accommodates drugs and a cover part 3' coupled to the body part 1'.

The body part 1' may include a drug accommodation part 11' having an internal space to accommodate the drugs, and the drug accommodation part 11' may include a drug discharge part 111' through which the drugs are discharged when the cover part 3' is removed.

Particularly, in the drop-type drug tube T' according to the related art, the body part 1' may be produced by injection molding, and the cover part 3' may be produced by injection molding separately form the body part 1'. After each of the body part 1' and the cover part 3 is injection-molded, the eye drops may be put through the drug discharge part 111' of the body part 1'.

However, the drug discharge part 111' may have a narrow discharge cross-sectional area so that a small amount of eye drops is discharged when used by a user in the future, and thus, it is inconvenient to accurately locate a drug injection equipment in the drug discharge part 111' and inject an accurate amount of eye drops, and as a result, many defective products have been generated in the corresponding process.

Particularly, when the plurality of drop-type drug tubes T' are placed, and simultaneously, the eye drops are put into the plurality of drop-type drug tubes T' through the drug injection equipment, difficulty in the process increases, and a limitation in which a plurality of defective products are generated has been occurred.

In addition, a process of bonding the body part 1' to the cover part 3' to supply a final product to the user may be performed. That is, after the drugs are injected through the drug discharge part 111', the drug discharge part 111' and the cover part 3' may be engaged with each other to perform the bonding process.

In the process of bonding the drug discharge part 111' to the cover part 3', an end of the drug discharge part 111' and the cover part 3' may be bonded, and thus, a space X' of an end of the drug discharge part 111' may not be used as a space for storing the eye drops to deteriorate space utilization efficiency.

Thus, research is being actively conducted on a structural design and manufacturing method of drop-type drug tubes that simplify the process and reduce the difficulty in the process.

US 2001/027301 A1 discloses a drop-type drug tube comprising a body part which defines an outer appearance and in which a drug is accommodated; and a cover part integrated with the body part and provided to be removed when the drug is used, wherein the body part comprises: a drug accommodation part having an internal space to accommodate the drug; a body support part provided to extend to the outside of the drug accommodation part; an upper insertion part and a lower insertion part; and the upper insertion part and the lower insertion part are bonded to each other in a state of being in contact with each other.

US 2003/146245 A1 discloses a drop dispenser comprising a hollow container body in which the liquid is contained and a dispensing nozzle unitary disposed with the container body, whereas the dispenser is formed by blow molding or vacuum forming an extruded parison within a hollow mold.

WO 2007/045360 A1 discloses a dispenser comprising a body part and a cover part which is removed when the formulation is used, the body part comprises a drug accommodation part and a body support part. The dispenser is formed by blow moulding process.

WO 02/074374 A1 discloses an ampoule comprising a body part and a cover part which is removed when the formulation is used, the body part comprises a drug accommodation part and a body support part. The ampoule body may be manufactured by blowing or vacuum forming the ampoule body in a mold. The ampoule body may then be filed with liquid, and a melt sealing process may be used to seal the body.

EP 1 075 875 A2 discloses a dispenser comprising a base part and a cover part which is removed when the formulation is used, the base part comprises a drug accommodation part and a body support part. The base part is formed by two-component injection moulding, in which different plastics or plastic variants for different areas of the shaped body are injected into the injection mold.

### SUMMARY

The present disclosure provides a method for manufacturing a drop-type drug tube having a structure with low processing difficulty, having maximized space utilization efficiency and having a structure that is conveniently used by a user.

The present disclosure provides a method for manufacturing a drop-type drug tube, which is capable of manufacturing the drop-type drug tube according to the above-described embodiments.

The present invention provides a method for manufacturing a drop-type drug tube with the features of the independent claim

Advantageous embodiments are subject matter of the dependent claims.

### BRIEF DESCRIPTION OF THE DRAWINGS

Exemplary embodiments can be understood in more detail from the following description taken in conjunction with the accompanying drawings, in which:
FIG. 1 is a view illustrating a drop-type drug tube according to a related art;
FIG. 2 is a perspective view of a drop-type drug tube according to an embodiment;
FIG. 3 is a view illustrating manufacturing of the drop-type drug tube according to an embodiment;
FIG. 4 is a view of an upper insertion part and a lower insertion part according to an embodiment;
FIG. 5 is a cross-sectional view of the drop-type drug tube according to an embodiment;
FIG. 6 is a cross-sectional view of a body part according to an embodiment;
FIG. 7 is a view illustrating a connection structure between the body part and a cover part according to an embodiment;
FIG. 8 is a flowchart illustrating a method for manufacturing a drop-type drug tube according to an embodiment;
FIG. 9 is a perspective view of a drop-type drug tube according to another embodiment;
FIG. 10 is a view illustrating manufacturing the drop-type drug tube according to another embodiment;
FIG. 11 is a perspective view of the drop-type drug tube in a state before a bonding process according to an embodiment;
FIG. 12 is a cross-sectional view of the drop-type drug tube according to another embodiment; and
FIG. 13 is a view illustrating a method for manufacturing a drop-type drug tube according to another embodiment.

### DETAILED DESCRIPTION OF EMBODIMENTS

Hereinafter, preferred embodiments of the present disclosure will be described in detail with reference to the accompanying drawings in such a manner that the technical idea of the present disclosure may easily be carried out by a person with ordinary skill in the art to which the invention pertains.

In the drawings, anything unnecessary for describing the present invention will be omitted for clarity, and also like reference numerals in the drawings denote like elements.

In this specification, duplicate descriptions of the same components are omitted.

In this specification, it will also be understood that when an element is referred to as being 'connected to' or 'coupled with' another element, it can be directly connected to the other element, or intervening elements may also be present. On the other hand, in this specification, it will be further understood that when one component is referred to as being 'directly connected' or 'directly linked' to another component, it means that no intervening component is present.

In addition, the terms used in this specification are merely used to describe specific embodiments.

In this specification, the terms of a singular form may include plural defines unless referred to the contrary.

In this application, the terms "comprises" or "having" are intended to indicate that there is a feature, number, step, operation, component, part, or combination thereof described in the specification, and one or more other features. It is to be understood that the present invention does not exclude the possibility of the presence or the addition of numbers, steps, operations, components, components, or a combination thereof.

In this specification, the term 'and/or' includes a combination of a plurality of listed items or any of the plurality of listed items. In this specification, 'A or B' may include 'A', 'B', or 'both A and B'.

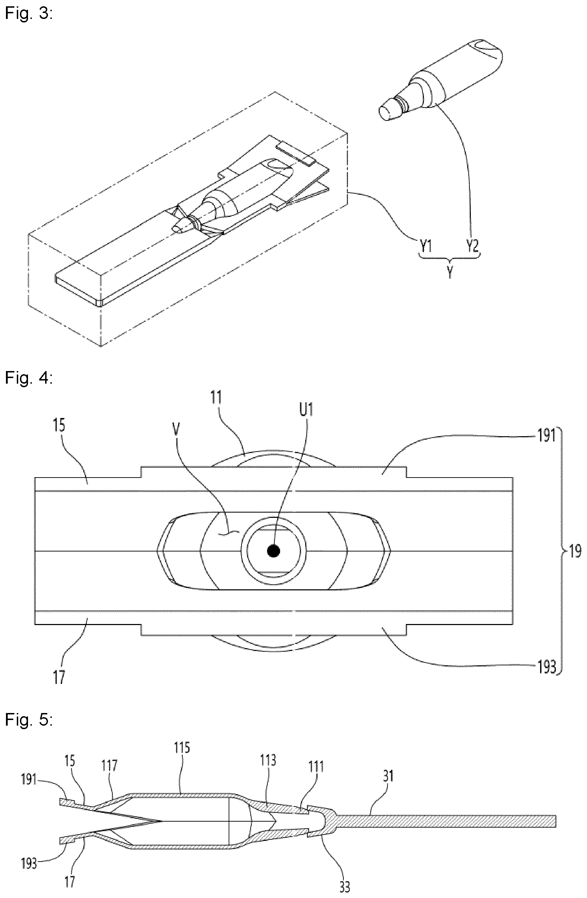

FIG. 2 is a perspective view of a drop-type drug tube according to an embodiment. FIG. 3 is a view illustrating manufacturing of the drop-type drug tube according to an embodiment. Referring to FIGS. 2 and 3, a drop-type drug tube T according to an embodiment may include a body part 1 and a cover part 3.

The body part 1 may define an outer appearance and accommodate a drug, and the cover part 3 may be integrated with the body part 1 and be provided to be removed when the drug is used. That is, the cover part 3 may be integrated with the body part 1 when a product is shipped and be configured to prevent the drug from being discharged to the outside, and a user may remove the cover part when using the product to use the drug in the body part 1.

The above-described drug may generally be eye drops such as artificial tears or eye water or may refer to liquid reagents.

In addition, the cover part 3 may be inserted into and coupled to the body part 1 when the drug remains after the user uses the drug to provide convenience of allowing the user to temporarily store the drug and then use the drug again.

In addition, since the cover part 3 is integrated with the body part 1 during injection molding, no separate bonding may be required to bond the cover part 3 to the body part 1, thereby reducing difficulty of a process.

Particularly, the body part 1 may include a drug accommodation part 11, a body support part 13, an upper insertion part 15, and a lower insertion part 17. The drug accommodation part 11 may have an internal space defined to accommodate the drug.

The body support part 13 may be provided to extend to the outside of the drug accommodation part 11, may improve durability when producing and distributing the product and when the product is used by the user, and may be easily held and used by the user.

The upper insertion part 15 may extend inclinedly upward from the drug accommodation part 11 and the body support part 13 so as to be away from the cover part 3. The lower insertion part 17 may face the upper insertion part 15 to extend inclinedly downward so as to be away from the cover part 3 in the drug accommodation part 11 and the body support part 13.

The upper insertion part 15 and the lower insertion part 17 may be provided symmetrically along a center line of the drug accommodation part 11, and the positions of the upper insertion part 15 and lower insertion part 17 may be changed depending on a viewing direction.

An internal space V may be defined by the injection molding in a state in which a mold Y is inserted between the upper insertion part 15 and the lower insertion part 17. In addition, after the mold Y is removed, the drug may be put into the internal space V, and the upper insertion part 15 and the lower insertion part 17 may be bonded to each other.

That is, unlike the related art, the drop-type drug tube T according to an embodiment may be manufactured by integrally injection-molding the body part 1 and the cover part 3, and thus, the manufacturing process may be more simplified compared to a case in which the body part 1 and the cover part 3 are separately injection-molded to be bonded to each other.

In addition, in the drop-type drug tube T according to an embodiment, the drug may be injected through a gap between the upper insertion part 15 and the lower insertion part 17 to solve a limitation in which it is difficult to put the drug through the drug discharge part, which will be described according to the related art, so as to accurately place a drug injection device in the drug discharge part 111 having a small area and also difficult to inject a fixed amount of the drug.

In addition, when compared to the prior art, a defect rate that occurs when the plurality of drop-type drug tubes T are simultaneously aligned and injected into the drug injection device may be visibly improved to simplify the process and improve process accuracy and economic efficiency.

In addition, the upper insertion part 15 may extend upward, and the lower insertion part 17 may extend downward. Thus, when the injection molding is performed, the mold V for providing the internal space V may efficiently move without an interference, and the drug injection device may efficiently move between the upper insertion part 15 and the lower insertion part 17 without an interference.

Furthermore, when the drug discharge part 111 and the cover part 3 are welded in the related art, there was high possibility of occurrence of defects due to a small welding area. However, in the drop-type drug tube T according to an embodiment, the upper insertion part 15 having a relatively wide area and the lower insertion part 17 may be bonded to each other to minimize defects, and when the bonding is performed, a distance from the drug may be maximized to maximally prevent the drug from being deformed due to heat or ultrasonic waves.

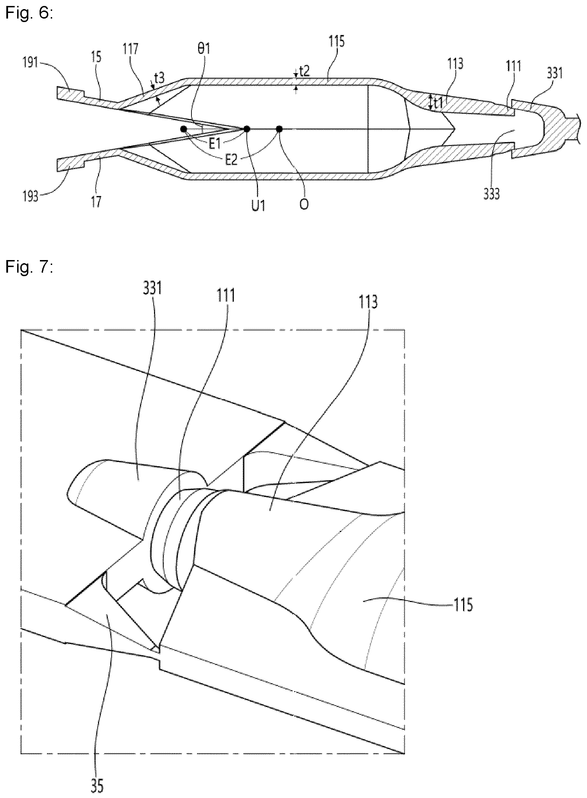

FIG. 4 is a view of the upper insertion part and the lower insertion part according to an embodiment. FIG. 5 is a cross-sectional view of the drop-type drug tube according to an embodiment. FIG. 6 is a cross-sectional view of the body part according to an embodiment.

Referring to FIGS. 4 to 6, the upper insertion part 15 and the lower insertion part 17 according to an embodiment may be angled at a predetermined angle. That is, the angle θ defined by the upper insertion part 15 and the lower insertion part 17 may be set at about 10 degrees to about 20 degrees.

That is, the mold Y may include a first mold Y1 that defines an overall appearance and a second mold Y2 that defines the internal space V, and the drop-type drug tube T may be manufactured through the injection molding in a state in which the first mold Y1 and the second mold A2 are disposed.

When the second mold Y2 that defines the internal space V is removed after molding the drop-type drug tube T, the upper insertion part 15 and the lower insertion part 17 may be angled at about 10 degrees to about 20 degrees so that the second mold Y2 is easily withdrawn.

In addition, the angle θ defined by the upper insertion part 15 and the lower insertion part 17 may be set at an angle of about 10 degrees to about 20 degrees so that, when the drug is injected into the internal space V, the drug may be prevented from flowing outward as much as possible.

Furthermore, the angle θ defined by the upper insertion part 15 and the lower insertion part 17 may be set at an angle of about 10 degrees to about 20 degrees so that the upper insertion part 15 and the lower insertion part 17 are easily bonded to each other in a state of being in contact with each other after the injection of the drug.

Particularly, the angle θ defined by the upper insertion part 15 and the lower insertion part 17 may be set at an angle of about 12.5 degrees to about 17.5 degrees. Thus, when the second mold Y2 defining the internal space V is removed after molding the drop-type drug tube T, the second mold Y2 may be withdrawn more easily.

In addition, the angle θ defined by the upper insertion part 15 and the lower insertion part 17 may be set at an angle of about 12.5 degrees to about 17.5 degrees so that, when the drug is injected into the internal space V, the drug may be more easily prevented from flowing outward.

More particularly, the angle θ defined by the upper insertion part 15 and the lower insertion part 17 may be set at an angle of about 14.5 degrees to about 15.5 degrees. Thus, when the second mold Y2 defining the internal space V is removed after molding the drop-type drug tube T, the second mold Y2 may be withdrawn more easily.

In addition, the angle θ defined by the upper insertion part 15 and the lower insertion part 17 may be set at an angle of about 14.5 degrees to about 15.5 degrees so that, when the drug is injected into the internal space V, the drug may be more easily prevented from flowing outward.

Referring again to FIGS. 5 and 6, the drug accommodation part 11 according to an embodiment may include a drug discharge part 111, a drug connection part 113, a drug accommodation body part 115, and a drug finish part 117.

The drug discharge part 111 may be integrated with the cover part 3, and the drug may be discharged when the cover part 3 is removed. That is, the drug discharge part 111 may be provided to have a diameter less than that of the drug accommodation body part 115, which will be described later, so that the drug is discharged consistently from the drug accommodation body part 115.

The drug connection part 113 may extend from the drug discharge part 111 so as to be away from the cover part 3 and may be provided to have an increasing diameter. That is, the drug connection part 113 may connect the drug discharge part 111to the drug accommodation body part 115.

When the drug directly flows from the drug accommodation body part 115 to the drug discharge part 111, the drug connection part 113 may suddenly increase in diameter and also may be provided to gradually increase in diameter toward the drug accommodation body part 115 so as to solve a limitation in which the drug is not properly discharged when the drug is discharged.

The drug accommodation body part 115 may extend from the drug connection part 113 so as to be away from the cover part 3 and may be provided to have a constant diameter. That is, the drug accommodation body part 115 may have the largest diameter and the largest area in the drug accommodation part 11 to effectively store the drug.

The drug finish part 117 may extend from the drug accommodation body part 115 to the cover part 3 and may be provided to decrease in diameter. That is, the drug finish part 117 may be provided to decrease in diameter in the drug accommodation body part 115 so that the upper insertion part 15 and the lower insertion part 17 are easily contacted with and bonded to each other.

That is, the drug accommodation part 11 may efficiently store the drug by varying in diameter of the drug discharge part 111, the drug connection part 113, the drug accommodation body part 115, and the drug finish part 117, may be easily used by the user, and may be configured so that products are easily manufactured.

The upper insertion part 15 may be provided to pass through the drug finish part 117 at a first point U of the drug accommodation body part 115, and the lower insertion part 17 may be provided to pass through the drug finish part 117 at the first point U of the drug accommodation body part 115.

That is, the upper insertion part 15 and the lower insertion part 17 may be provided symmetrically with respect to a center line of the drug accommodation body part 115 and may be provided to pass through the entire drug finish part 117 at one side of the drug accommodation body part 115.

Thus, when the second mold Y2 defining the internal space V is removed after molding the drop-type drug tube T, the second mold Y2 may be withdrawn more easily, and when the drug is injected, the drug may be easily prevented from flow to the outside.

In addition, when the first mold Y1 and the second mold Y2 are removed after molding the drop-type drug tube T, the overall appearance of the drop-type drug tube T may be effectively maintained to minimize a defect rate.

Particularly, a distance E1 from the drug finish part 117 to the first point U may range of about 0.7 and about 0.9 of a distance E2 from the drug finish part 117 to a center of the drug accommodation body part 115.

Thus, when the second mold Y2 defining the internal space V is removed after molding the drop-type drug tube T, the second mold Y2 may be withdrawn more easily, and when the drug is injected, the drug may be easily prevented from flowing to the outside.

In addition, when the first mold Y1 and the second mold Y2 are removed after molding the drop-type drug tube T, the overall appearance of the drop-type drug tube T may be effectively maintained to further minimize the defect rate.

More particularly, the distance E1 from the drug finish part 117 to the first point U may be about 0.75 times and about 0.85 times the distance E2 from the drug finish part 117 to the center of the drug accommodation body part 115.

Thus, when the second mold Y2 defining the internal space V is removed after molding the drop-type drug tube T, the second mold Y2 may be withdrawn more easily, and when the drug is injected, the drug may be easily prevented from flowing to the outside.

In addition, when the first mold Y1 and the second mold Y2 are removed after molding the drop-type drug tube T, the overall appearance of the drop-type drug tube T may be effectively maintained to further minimize the defect rate.

A thickness t1 of the drug connection part 113 may be greater than each of a thickness t2 of the drug accommodation body part 115 and a thickness t3 of the drug finish part 117. That is, the drug connection part 113 may connect the drug accommodation body part 115 to the drug discharge part 111 and may be provided with a relatively large thickness as a flow path through which the drug is discharged when the user uses the drug to improve structural safety.

Particularly, the thickness t2 of the drug accommodation body part 115 and the thickness t3 of the drug finish part 117 may be provided to be the same, and the thickness t1 of the drug connecting portion 113 may be provided to be about 2 times to about 4 times the thickness t2 of the drug accommodation body part 115.

Since the drug accommodation body part 115 and the drug finish part 117 are not the path through which the drug is discharged when the user uses the drug, each of the drug accommodation body part 115 and the drug finish part 117 may have a relatively small thickness to maximally store the drug, thereby maximizing storage capacity and securing structural safety above a certain level.

The thickness t1 of the drug connection part 113 may be about 2 times to about 4 times the thickness t2 of the drug accommodation body part 115 to more efficiently secure the structural safety when the drug is used, thereby effectively securing the overall storage capacity and user's feeling of use of the user.

The body part 1 according to an embodiment may include an insertion support part 19. The insertion support part 19 may include a first insertion support part 191 and a second insertion support part 193. The first insertion support part 191 may protrude upward from a distal end of the upper insertion part 15, and the second insertion support part 193 may protrude downward from a distal end of the lower insertion part 17.

In addition, the first insertion support part 191 may be provided at a center of the upper insertion part 15, and the second insertion support part 193 may be provided at a center of the lower insertion part 17, and the first insertion support part 191 and the second insertion support part 193 may be provided to be symmetrical to each other.

When the second mold Y2 moves between the upper insertion part 15 and the lower insertion part 17, structural rigidity of a portion at which the upper insertion part 15 and the lower insertion part 17 are in contact with each other may increase by the first insertion support part 191 and the second insertion support part 193 to maximally prevent tearing and damage from occurring.

In addition, the first insertion support part 191 may be provided at a portion of the center rather than the entire end of the upper insertion part 15, and the second insertion support part 193 may be provided not on the entire end of the lower insertion part 17, but a portion of the center to minimize an increase in weight or volume of the entire drop-type drug tube T while effectively securing the structural safety.

FIG. 7 is a view illustrating a connection structure between the body part and the cover part according to an embodiment. Referring to FIGS. 5 to 7, the cover part 3 according to an embodiment may include a cover body part 31, a cover protrusion 33, and a cover removing part 35.

The cover body part 31 may be provided in a plate shape and may be spaced apart from the body support part 13. That is, the cover body part 31 may be provided in a plate shape so that the user easily holds the cover body part 31 when removing the cover part 3, and the body support part 13 may also be provided in a plate shape so that the user holds the cover body part 31 to easily remove the cover part 3.

In addition, a thickness of the cover body part 31 may be greater than that of the body support part 13. That is, the body support part 13 may be connected to the drug accommodation part 11 in which the drug is accommodated to secure the structural safety above a certain level. However, since the cover body part 31 occupies most of an area of the cover part 3, the user may easily hold the cover body part 31 when removing the cover part 3, and the cover part 31 may be provided to be larger than the body support part 13 to improve the structural safety.

Particularly, the thickness of the cover body part 31 may be about 1.1 times to about 1.3 times the thickness of the body support part 13. That is, the thickness of the cover body part 31 may be about1.1 times to about 1.3 times the thickness of the body support part 13, and thus, the user may easily hold the cover body part 31 when removing the cover body part 31 to effectively secure the structural safety.

More particularly, the thickness of the cover body part 31 may be about 1.15 times to about 1.25 times the thickness of the body support part 13. That is, the thickness of the cover body part 31 may be about 1.15 times to about 1.35 times the thickness of the body support part 13, and thus, the user may easily hold the cover body part 31 when removing the cover body part 31 to more effectively secure the structural safety.

The cover protrusion 33 may be provided to protrude to a shape corresponding to that of the drug discharge part 111 on one surface of the cover body part 31, which faces the drug discharge part 111. That is, the cover protrusion 33 may have the same shape as the drug discharge part 111 and may have a cross-section greater than that of the drug discharge part 111.

Thus, the user may effectively remove the cover part 3, use the drug, and then close the drug discharge part 111 again through the cover protrusion 33 to enable temporary storage.

The cover removing part 35 may extend from the cover body part 31 to both ends of the body support part 13 and may be provided to increase in cross-sectional area as it moves from the cover body part 31 to the body support part 13. That is, the cover removing part 35 may be a portion that is directly connected to the body part 1, and the cover body part 31 may be provided to a predetermined thickness or more to secure the structural safety so that the user removes the cover part 3 from the body part 1.

Particularly, the cover removing part 35 may extend to decrease in cross-sectional area from each of upper and lower ends of the cover body part 31 toward the center of the body support part 13. Thus, the user may easily remove the cover part 3 from the body part 1.

The cover protrusion 33 may include a cover protrusion body part 331 and a cover protrusion central part 333. The cover protrusion body part 331 may have an inner diameter greater than an outer diameter of the drug discharge part 111. That is, the cover protrusion body part 331 may be provided so that an inner circumference thereof is in contact with only an outer circumference of the drug discharge part 111 and may be easily separated when removed by the user.

In addition, after the user uses the drop-type drug tube T, the cover protrusion body part 331 may be inserted to surround an outer circumferential surface of the drug discharge part 111, thereby effectively temporarily storing the drug.

The cover protrusion central part 333 may be provided to be recessed in a direction that is away from the drug discharge part 111 in one surface of the cover protrusion body part 331, which faces the drug discharge part 111. That is, the cover protrusion central part 333 may store the drug together with the drug discharge part 111 as much as the recessed space to maximize the overall capacity of the drop-type drug tube T.

In addition, when the drug is injected between the upper insertion part 15 and the lower insertion part 17, even though air enters together to occupy a portion the internal space V, the drug stored in the cover protrusion central part 333 may supplement the space.

In addition, when the cover protrusion central part 333 is inserted so that the outer circumferential surface of the drug discharge part 111 is surrounded by the cover protrusion body part 331 after the user uses the drop-type drug tube T, the cover protrusion central part 333 may close the drug discharge part 111 to maximally prevent the drug from leaking to the outside.

That is to say, unlike the related art, in the drip-type drug tube T according to an embodiment, the cover protrusion 33 and the drug discharge part 111 may not be bonded to each other to maximally utilize the space of the drug discharge part 111.

In addition, since there is no bonding between the cover protrusion part 33 and the drug discharge part 111, the cover protrusion central part 333 may be formed to store the drug in the cover protrusion central part 333 together with the drug discharge part 111. That is, the space utilization of the drop-type drug tube T through the cover protrusion central part 333 may be maximized.

An outer diameter D1 of the cover protrusion body part 331 may be about 1.1 times to about 1.3 times an outer diameter D2 of the drug discharge part 111. That is, the cover protrusion body part 331 has an outer diameter D1 that is about 1.1 times to about 1.3 times the outer diameter D2 of the drug discharge part 111 to minimize the volume and weight of the product while maximally securing the structural safety during the injection molding and the product manufacturing process after the injection molding.

In addition, the cover protrusion body part 331 may be provided to have an outer diameter D1 that is about 1.1 times to about 1.3 times the outer diameter D2 of the drug discharge part 111, and thus, the user may easily remove the cover part 3 when using the drug.

Particularly, the outer diameter D1 of the cover protrusion body part 331 may be about 1.15 times to about 1.25 times the outer diameter D2 of the drug discharge part 111. Thus, the structural safety may be further secured as much as possible during the injection molding and the post-injection molding product manufacturing process to further minimize the volume and weight of the product.

In addition, the user may more easily remove the cover part 3 when using the drugs.

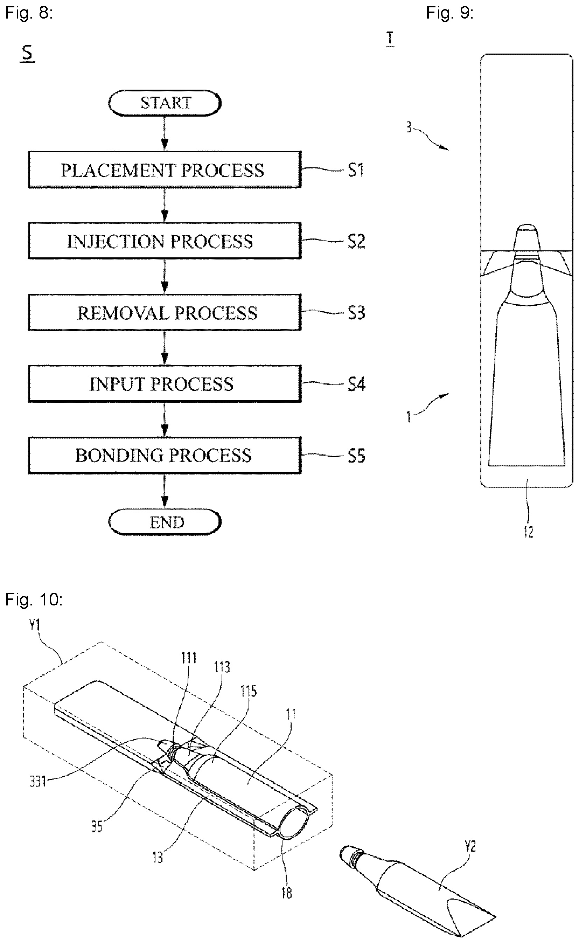

FIG. 8 is a flowchart illustrating a method for manufacturing a drop-type drug tube according to an embodiment. A method (S) for manufacturing a drop-type drug tube according to an embodiment may include a placement process (S1), an injection process (S2), a removal process (S3), an input process (S4), and a bonding process (S5).

That is, a method (S) for manufacturing the above-described drop-type drug tube T may include a placement process (S1), an injection process (S2), a removal process (S3), an input process (S4), and a bonding process (S5). In the placement process (S1), a first mold Y1 for integrally forming an outer appearance of a body part 1 and a cover part 3 and a second mold Y2 for forming an internal space V of a drug accommodation part 11 may be disposed.

In the injection process (S2), a molding material may be injected between the first mold Y1 and the second mold Y2. That is, the injection process S2 may be a type of injection molding that involves a process of injection molding an existing plastic injection product.

In the removal process (S3), the second mold Y2 may be removed between an upper insertion part 15 and a lower insertion part 17. That is, in the removal process (S3), the second mold Y2 may be easily removed due to the above-described structural features of the upper insertion part 15 and the lower insertion part 17.

In addition, the second mold Y2 may have an inner portion inserted into an internal space V having a cross-sectional area less than that of an outer portion so s to be effectively removed after the injection molding. Thus, the injection molding may be easily performed, the removal after the injection molding may be easily performed, and damage to the drop-type drug tube T may be minimized.

In the input process (S4), the drug may be injected between the upper insertion part 15 and the lower insertion part 17. That is, in the input process (S4), the drug may be easily input due to the above-described structural features of the upper insertion part 15 and the lower insertion part 17.

In the bonding process (S5), the upper insertion part 15 and the lower insertion part 17 may be bonded to each other. That is, the bonding process (S5) may be performed by heat welding or ultrasonic waves along edges of the upper insertion part 15 and the lower insertion part 17.

Thus, unlike the welding between the drug discharge part 111 and the cover part 3 according to the related art, the drug discharge part 111 and the cover part 3 may be integrated with each other to maximally utilize a space between the drug discharge part 111 and the cover part 3, thereby improvising the space utilization.

In addition, unlike the related art, in which the drug is injected through the drug discharge part 111, making it difficult to align a position of the drug injection device and inject a fixed amount, the drug may be injected between the upper insertion part 15 and the lower insertion part 17, each of which has a wide area, so that the alignment and the quantitative input are easy. Particularly, it is more effective when injecting the drug into the plurality of drop-type drug tubes T at once.

The drop-type drug tube T according to an embodiment may be made of a material containing about 50% ultra-high molecular weight polyethylene (UHMWPE). That is, each of the body part 1 and the cover part 3 may be made of a material containing 50% ultra-high molecular weight polyethylene (UHMWPE).

UHMWPE may have excellent moisture resistance, wear resistance, and resistance to various organic solvents, strong acids, and strong alkalis, and may be often used instead of steel (especially wire). Since UHMWPE is much lighter than steel and floated on water, UHMWPE may be widely used in ship cables and fishing lines.

Particularly, steel cables are very dangerous if cut while stretched (a person may be died if hit), but UHMWPE cables may be light, and thus, even if a person is hit by the cut cable, the person may not be seriously injured. In addition, UHMWPE may be advantage that it is much cheaper and easier to be manufactured than carbon fiber.

That is, each of the body part 1 and the cover part 3 may be made of a material containing 50% UHMWPE, and thus, the drug inside the drug tube may be prevented being evaporated as much as possible even when stored for a long period of time.

FIG. 9 is a perspective view of a drop-type drug tube according to another embodiment. FIG. 10 is a view illustrating manufacturing the drop-type drug tube according to another embodiment. Referring to FIGS. 9 and 10, a drop-type drug tube T according to another embodiment may include a body part 1 and a cover part 3.

Hereinafter, descriptions of contents that are duplicated with the contents described above will be omitted, and the explanation will focus on the newly added configuration. However, the contents described above should not be excluded from the scope of rights or interpreted as limited.

The body part 1 may define an outer appearance and accommodate a drug, and the cover part 3 may be integrated with the body part 1 and be provided to be removed when the drug is used. Particularly, the body part 1 may include a drug accommodation part 11, a body support part 13, and a mold insertion part 18.

The drug accommodation part 11 may have an internal space defined to accommodate the drug. The body support part 13 may be provided to extend to the outside of the drug accommodation part 11, may improve durability when producing and distributing the product and when the product is used by the user, and may be easily held and used by the user.

The mold insertion part 18 may be provided at an end of the drug accommodation part 11, and thus, a mold Y may be inserted therein. In addition, injection molding may be performed in the state in which the mold is inserted into the mold insertion part 18 to form an internal space V. After the mold is removed, the drug may be put into the internal space V, and the mold insertion part 18 may be bonded.

That is, the mold Y may be withdrawn through the mold insertion part 18, and the drop-type drug tube T may be manufactured by bonding the end of the mold insertion part 18. Particularly, the mold insertion part 18 may be bonded only in a direction perpendicular to a direction in which the drug accommodation part 11 moves away from the cover part 3.

Thus, the mold insertion part 18 may minimize the bonding process when manufacturing the drop-type drug tube T to improve manufacturing efficiency. In addition, since the bonding is performed only at a lower end of the mold insertion part 18, a bonded area may be minimized to minimize leakage of the drug.

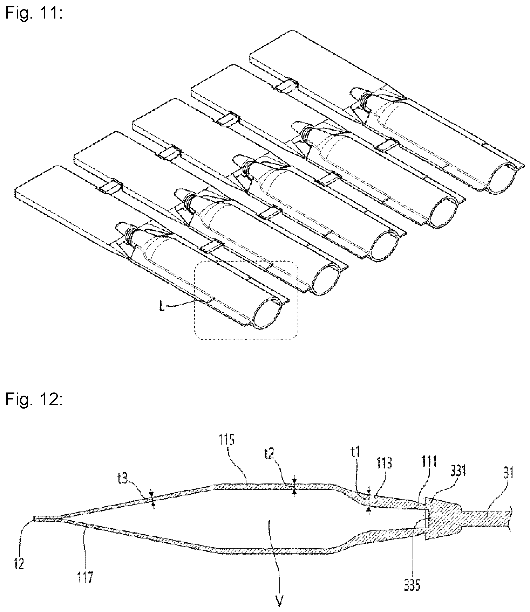

FIG. 11 is a perspective view of the drop-type drug tube in a state before the bonding process (S5) according to an embodiment.

The bonding process (S5) may be a process of bonding top and bottom surfaces of the mold insertion part 18 through thermal or ultrasonic welding, e.g., a process of fusing a lower end of a bonding line L. FIG. 12 is a cross-sectional view of the drop-type drug tube according to another embodiment. Referring again to FIG. 12, the drug accommodation part 11 according to another embodiment may include a drug discharge part 111, a drug connection part 113, a drug accommodation body part 115, and a drug finish part 117.

The drug discharge part 111 may be integrated with the cover part 3, and the drug may be discharged when the cover part 3 is removed. That is, the drug discharge part 111 may be provided to have a diameter less than that of the drug accommodation body part 115, which will be described later, so that the drug is discharged consistently from the drug accommodation body part 115.

The drug connection part 113 may extend from the drug discharge part 111 so as to be away from the cover part 3 and may be provided to have an increasing diameter. That is, the drug connection part 113 may connect the drug discharge part 111to the drug accommodation body part 115.

When the drug directly flows from the drug accommodation body part 115 to the drug discharge part 111, the drug connection part 113 may suddenly increase in diameter and also may be provided to gradually increase in diameter toward the drug accommodation body part 115 so as to solve a limitation in which the drug is not properly discharged when the drug is discharged.

The drug accommodation body part 115 may extend from the drug connection part 113 so as to be away from the cover part 3 and may be provided to have a constant diameter. That is, the drug accommodation body part 115 may have the largest diameter and the largest area in the drug accommodation part 11 to effectively store the drug.

The drug finish part 117 may extend from the drug accommodation body part 115 to the cover part 3 and may be provided to decrease in diameter. In addition, the drug finish part 117 may be provided by bonding the mold insertion part 18. That is, the drug finish part 117 may be provided to decrease in diameter as it moves away from the drug accommodation body part 115 as a pressure is applied while the mold insertion part 18 is bonded.

That is, the drug accommodation part 11 may efficiently store the drug by varying in diameter of the drug discharge part 111, the drug connection part 113, the drug accommodation body part 115, and the drug finish part 117, may be easily used by the user, and may be configured so that products are easily manufactured.

In addition, a thickness t1 of the drug connection part 113 may be greater than each of a thickness t2 of the drug accommodation body part 115 and a thickness t3 of the drug finish part 117. That is, the drug connection part 113 may connect the drug accommodation body part 115 to the drug discharge part 111 and may be provided with a relatively large thickness as a flow path through which the drug is discharged when the user uses the drug to improve structural safety.

Particularly, the thickness t2 of the drug accommodation body part 115 may be provided to be greater than the thickness t3 of the drug finish part 117, and the thickness t1 of the drug connecting portion 113 may be provided to be about 2 times to about 4 times the thickness t2 of the drug accommodation body part 115.

Since the drug accommodation body part 115 is not the path through which the drug is discharged when the user uses the drug, each of the drug accommodation body part 115 may have a relatively small thickness to maximally store the drug, thereby maximizing storage capacity and securing structural safety above a certain level.

In addition, in the case of the drug finish part 117, the thickness may be relatively less than that of the drug accommodation body part 115 due to compression during manufacturing to secure structural safety above a certain level while maximizing storage capacity.

The thickness t1 of the drug connection part 113 may be about 2 times to about 4 times the thickness t2 of the drug accommodation body part 115 to more efficiently secure the structural safety when the drug is used, thereby effectively securing the overall storage capacity and user's feeling of use of the user.

The drop-type drug tube T according to another embodiment may further include a body finish part 12. The body finish part 12 may be connected to the drug finish part 117 and the body support part 13 and may be provided to have a diameter less than that of the drug finish part 117.

In addition, the body finish part 12 may be provided by bonding the mold insertion part 18. That is, the body finish part 12 may be provided by compression during the process of bonding the mold insertion part 18. Thus, the process of manufacturing the drop-type drug tube T may be simplified, and the bonding area may be minimized to reduce possibility of leakage of the drug. Furthermore, the body finish part 12 may also be formed by compressing an end of the body support part 13 when bonding and compressing the mold insertion part 18.

The cover part 3 according to another embodiment may include a cover body part 31, a cover protrusion 33, and a cover removing part 35.

The cover body part 31 may be provided in a plate shape and may be spaced apart from the body support part 13. That is, the cover body part 31 may be provided in a plate shape so that the user easily holds the cover body part 31 when removing the cover part 3, and the body support part 13 may also be provided in a plate shape so that the user holds the cover body part 31 to easily remove the cover part 3.

In addition, a thickness of the cover body part 31 may be greater than that of the body support part 13. That is, the body support part 13 may be connected to the drug accommodation part 11 in which the drug is accommodated to secure the structural safety above a certain level. However, since the cover body part 31 occupies most of an area of the cover part 3, the user may easily hold the cover body part 31 when removing the cover part 3, and the cover part 31 may be provided to be larger than the body support part 13 to improve the structural safety.

The cover protrusion 33 may be provided to protrude to a shape corresponding to that of the drug discharge part 111 on one surface of the cover body part 31, which faces the drug discharge part 111. That is, the cover protrusion 33 may have the same shape as the drug discharge part 111 and may have a cross-section greater than that of the drug discharge part 111.

Thus, the user may effectively remove the cover part 3, use the drug, and then close the drug discharge part 111 again through the cover protrusion 33 to enable temporary storage.

The cover removing part 35 may extend from the cover body part 31 to both ends of the body support part 13 and may be provided to increase in cross-sectional area as it moves from the cover body part 31 to the body support part 13. That is, the cover removing part 35 may be a portion that is directly connected to the body part 1, and the cover body part 31 may be provided to a predetermined thickness or more to secure the structural safety so that the user removes the cover part 3 from the body part 1.

The cover protrusion 33 may include a cover protrusion body part 331 and a cover protrusion closing part 335. The cover protrusion body part 331 may have an inner diameter greater than an outer diameter of the drug discharge part 111. In addition, the cover protrusion closing portion 335 may be provided on one surface of the cover protrusion body part 331 facing the drug discharge part 111 and may extend parallel to the drug discharge part 111.

That is, the cover protrusion closing part 335 may be connected to an end of the drug discharge part 111 from an inner circumferential surface of the cover protrusion body part 331 to close an opening of the drug discharge part 111. Thus, structural safety of the drip-type drug tube T according to another embodiment may be maximized to maximally prevent the drug from leaking between the cover part 3 and the body part 1.

The drop-type drug tube T according to another embodiment may be made of a material containing about 50% ultra-high molecular weight polyethylene (UHMWPE). That is, each of the body part 1 and the cover part 3 may be made of a material containing 50% ultra-high molecular weight polyethylene (UHMWPE).

UHMWPE may have excellent moisture resistance, wear resistance, and resistance to various organic solvents, strong acids, and strong alkalis, and may be often used instead of steel (especially wire). Since UHMWPE is much lighter than steel and floated on water, UHMWPE may be widely used in ship cables and fishing lines.

Particularly, steel cables are very dangerous if cut while stretched (a person may be died if hit), but UHMWPE cables may be light, and thus, even if a person is hit by the cut cable, the person may not be seriously injured. In addition, UHMWPE may be advantage that it is much cheaper and easier to be manufactured than carbon fiber.

That is, each of the body part 1 and the cover part 3 may be made of a material containing 50% UHMWPE, and thus, the drug inside the drug tube may be prevented being evaporated as much as possible even when stored for a long period of time.

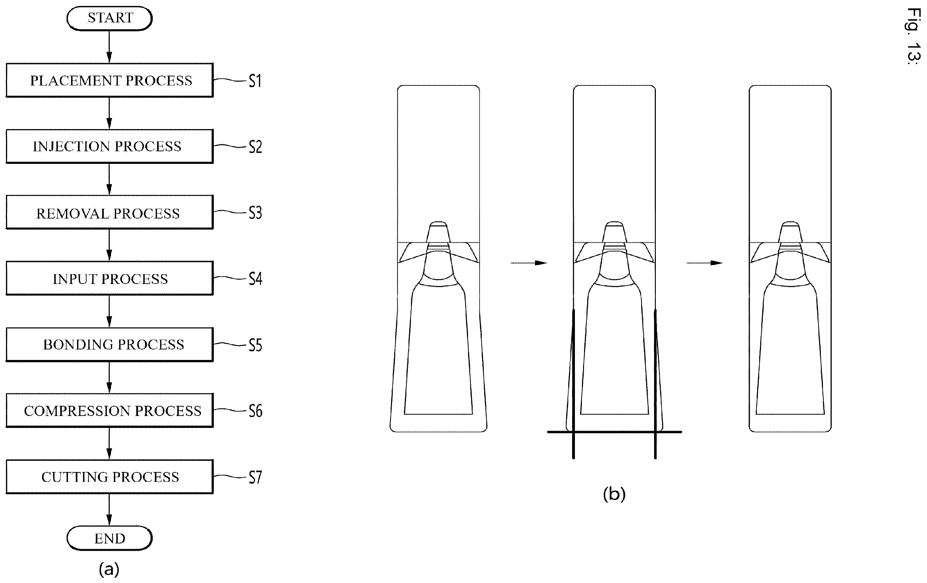

FIG. 13 is a view illustrating a method for manufacturing a drop-type drug tube according to another embodiment. Particularly, FIG. 13(a) is a flowchart illustrating a method for manufacturing a drop-type drug tube according to another embodiment, and FIG. 13(b) is a flowchart illustrating bonding and cutting processes of the drop-type drug tube according to another embodiment.

Referring to FIG. 13, a method (S) for manufacturing a drop-type drug tube according to another embodiment may include a placement process (S1), an injection process (S2), a removal process (S3), an input process (S4), a bonding process (S5), a compression process (S6), and a cutting process (S7).

That is, a method (S) for manufacturing the above-described drop-type drug tube T may include a placement process (S1), an injection process (S2), a removal process (S3), an input process (S4), and a bonding process (S5). In the placement process (S1), a first mold Y1 for integrally forming an outer appearance of a body part 1 and a cover part 3 and a second mold Y2 for forming an internal space V of a drug accommodation part 11 may be disposed.

In the injection process (S2), a molding material may be injected between the first mold Y1 and the second mold Y2. That is, the injection process S2 may be a type of injection molding that involves a process of injection molding an existing plastic injection product.

In the removal process (S3), the second mold Y2 may be removed from the mold insertion part 18. That is, in the removal process (S3), the second mold Y2 may be easily removed due to its structural feature in which only the second mold Y2 needs to be removed from the mold insertion part 18.

In the input process (S4), the drug may be input into the mold insertion part 18. That is, in the input process (S4), the drug may be easily input due to the simple structural features of the mold insertion part 18.

In the bonding process (S5), the mold insertion part 18 may be bonded. That is, in the bonding process (S5), upper and lower portions of the mold insertion part 18 may be bonded through heat welding or ultrasonic welding.

In the compression process (S6), the mold insertion part 18 and the body support part 13, which are bonded in the bonding process (S5), may be compressed to increase bonding force of the mold insertion part 18, and thus, the entire outer appearance of the drop-type drug tube T may be provided aesthetically.

In the cutting process (S7), the body support part 13 may protrude outward through the bonding process (S5) and the compression process (S6), and then, the protruding portion may be cut. Thus, the overall appearance of the drop-type drug tube T may be provided aesthetically.

Embodiments may provide the drop-type drug tube having the structure with the low processing difficulty.

Embodiments may also provide the drop-type drug tube having the maximized space utilization efficiency.

Embodiments may also provide the drop-type drug tube having the structure that is conveniently used by the user.

Embodiments may also provide the drop-type drug tube, which is capable of manufacturing the drop-type drug tube according to the above-described embodiments.

Although the present invention has been described in detail through exemplary embodiments above, those of ordinary skill in the art to which the present invention pertains will understand that various modifications can be made to the above-described embodiments.

Therefore, the scope of this disclosure is defined not by the detailed description of the invention but by the appended claims.

## Claims

1. A method for manufacturing a drop-type drug tube;
the drop-type drug tube (T) comprising:
a body part (1) which defines an outer appearance and in which a drug is accommodated; and
a cover part (3) integrated with the body part (1) and provided to be removed when the drug is used,
wherein the body part (3) comprises:
a drug accommodation part (11) having an internal space (V) to accommodate the drug;
a body support part (13) provided to extend to the outside of the drug accommodation part (11);
an upper insertion part (15) inclinedly extending upward from the drug accommodation part (11) and the body support part (13) so as to be away from the cover part (3); and
a lower insertion part (17) provided to face the upper insertion part (15) and inclinedly extending downward from the drug accommodation part (11) and the body support part (13) so as to be away from the cover part (3),
the method (S) comprising:
a placement process (S1) of disposing a first mold for integrally forming an outer appearance of the cover part together with the body part and a second mold for forming the internal space of the drug accommodation part;
an injection process (S2) of inputting a molding material between the first mold and the second mold;
a removal process (S3) of removing the second mold between the upper insertion part and the lower insertion part;
an input process (S4) of inputting the drug between the upper insertion part and the lower insertion part; and
a bonding process (S5) of bonding the upper insertion part to the lower insertion part
wherein injection molding (S2) is performed in a state in which a mold is injected between the upper insertion part (15) and the lower insertion part (17) to define the internal space (V), and
after the mold is removed, the drug is put into the internal space (V), and the upper insertion part (15) and the lower insertion part (13) are bonded to each other.

2. The method of claim 1, wherein an angle (θ) between the upper insertion part and the lower insertion part is set to about 10 degrees to about 20 degrees.

3. The methodof claim 2, wherein the angle (θ) between the upper insertion part and the lower insertion part is set to about 12.5 degrees to about 17.5 degrees.

4. The methodof claim 3, wherein the drug accommodation part (11) comprises:
a drug discharge part (111) which is integrated with the cover part and through which the drug is discharged when the cover part is removed;
a drug connection part (113) extending from the drug discharge part so as to be away from the cover part and having an increasing diameter;
a drug accommodation body part (115) extending from the drug discharge part so as to be away from the cover part and having a constant diameter; and
a drug finish part (117) extending from the drug discharge part so as to be away from the cover part and having an decreasing diameter,
wherein the upper insertion part (15) is provided to pass through the drug finish part at a first point of the drug accommodation body part, and
the lower insertion part (17) is provided to pass through the drug finish part at the first point of the drug accommodation body part,
wherein the first point is disposed closer to the drug finish part than a center of the drug accommodation body part.

5. The method of claim 4, wherein a distance (E1) from the drug finish part to the first point is about 0.7 times to about 0.9 times a distance (E2) from the drug finish part to the center of the drug accommodation body part.

6. The method of claim 4, wherein a distance (E1) from the drug finish part to the first point is about 0.75 times to about 0.85 times a distance (E2) from the drug finish part to the center of the drug accommodation body part.

7. The method of claim 6, wherein a thickness (t1) of the drug connection part is greater than each of a thickness (t2) of the drug accommodation body part and a thickness (t3) of the drug finish part.

8. The method of claim 7, wherein the thickness (t2) of the drug accommodation body part and the thickness (t3) of the drug finish part are the same, and
the thickness (t1) of the drug connection part is about 2 times to about 4 times the thickness (t2) of the drug accommodation body part.

9. The method of claim 8, wherein the body part further comprises an insertion support part comprising a first insertion support part protruding upward from a distal end of the upper insertion part and a second insertion support part protruding downward from a distal end of the lower insertion part.

10. The method of claim 9, wherein the cover part (3) comprises:
a cover body part (31) provided in a plate shape and disposed to be spaced apart from the body support part;
a cover protrusion (33) protruding in a shape corresponding to that of the drug discharge part on one surface of the cover body part, which faces the drug discharge part; and
a cover removing part (35) extending from the cover body part to both ends of the body support part and provided to have a cross-sectional area that gradually decreases from the cover body part to the body support part,
wherein the cover protrusion (33) comprises:
a cover protrusion body part having an inner diameter greater than an outer diameter of the drug discharge part; and
a cover protrusion central part provided to be recessed in a direction away from the drug discharge part on one surface of the cover protrusion body part, which faces the drug discharge part.

11. The method of claim 10, wherein an outer diameter (D1) of the cover protrusion body part is about 1.1 times to about 1.3 times an outer diameter (D2) of the drug discharge part.

## Patentansprüche

1. Ein Verfahren zur Herstellung eines Medikamentenröhrchens vom Tropfen-Typ;
das Medikamentenröhrchens vom Tropfen-Typ (T) umfassend:
einen Körperteil (1), der eine äußere Erscheinung definiert und in dem ein Medikament aufgenommen ist; und
einen Abdeckteil (3), der mit dem Körperteil (1) integriert ist und dazu vorgesehen ist, entfernt zu werden, wenn das Medikament verwendet wird,
wobei der Körperteil (3) umfasst:
einen Medikamentenaufnahmeteil (11) mit einem Innenraum (V) zur Aufnahme des Medikaments;
einen Körperstützteil (13), der dazu vorgesehen ist, sich zur Außenseite des Medikamentenaufnahmeteils (11) zu erstrecken
ein oberer Einführteil (15), der sich von dem Medikamentenaufnahmeteil (11) und dem Körperstützteil (13) schräg nach oben erstreckt, so dass er von dem Abdeckteil (3) entfernt ist; und
ein unterer Einführteil (17), der dazu vorgesehen ist, dem oberen Einführteil (15) zugewandt zu sein, und der sich von dem Medikamentenaufnahmeteil (11) und dem Körperstützteil (13) schräg nach unten erstreckt, so dass er von dem Abdeckteil (3) entfernt ist,
das Verfahren (S) umfassend:
einen Platzierungsvorgang (S1) des Anordnens einer ersten Form zum integralen Bilden einer äußeren Erscheinung des Abdeckteils zusammen mit dem Körperteil und einer zweiten Form zum Bilden des Innenraums des Medikamentenaufnahmeteils;
einen Einspritzvorgang (S2) zum Eingeben eines Formmaterials zwischen die erste Form und die zweite Form;
einen Entfernungsvorgang (S3) zum Entfernen der zweiten Form zwischen dem oberen Einführteil und dem unteren Einführteil;
einen Eingabevorgang (S4) zum Eingeben des Medikaments zwischen dem oberen Einführteil und dem unteren Einführteil; und
einen Verbindungsvorgang (S5) zum Verbinden des oberen Einführteils mit dem unteren Einführteil
wobei das Spritzgießen (S2) in einem Zustand durchgeführt wird, in dem eine Form zwischen dem oberen Einführteil (15) und dem unteren Einführteil (17) eingespritzt ist, um den Innenraum (V) zu definieren, und
nach dem Entfernen der Form wird das Medikament in den Innenraum (V) gegeben, und der obere Einführteil (15) und der untere Einführteil (13) werden miteinander verbunden.

2. Das Verfahren nach Anspruch 1, wobei ein Winkel (θ) zwischen dem oberen Einführteil und dem unteren Einführteil auf etwa 10 Grad bis etwa 20 Grad festgelegt wird.

3. Das Verfahren nach Anspruch 2, bei dem der Winkel (θ) zwischen dem oberen Einführteil und dem unteren Einführteil auf etwa 12,5 Grad bis etwa 17,5 Grad festgelegt wird.

4. Das Verfahren nach Anspruch 3, wobei das Medikamentenaufnahmeteil (11) umfasst:
einen Medikamentenabgabeteil (111), der mit dem Abdeckteil integriert ist und durch den das Medikament abgegeben wird, wenn das Abdeckteil entfernt ist;
einen Medikamentenverbindungsteil (113), der sich von dem Medikamentenabgabeteil so erstreckt, dass er von dem Abdeckteil entfernt ist, und der einen zunehmenden Durchmesser aufweist;
einen Medikamentenaufnahmekörperteil (115), der sich von dem Medikamentenabgabeteil so erstreckt, dass er von dem Abdeckteil entfernt ist, und der einen konstanten Durchmesser aufweist; und
einen Medikamentenabschlussteil (117), der sich von dem Medikamentenabgabeteil so erstreckt, dass er von dem Abdeckteil entfernt ist, und einen abnehmenden Durchmesser aufweist,
wobei das obere Einführteil (15) dazu vorgesehen ist, durch den Medikamentenabschlussteil an einem ersten Punkt des Medikamentenaufnahmekörperteils zu verlaufen, und
das untere Einführteil (17) dazu vorgesehen ist, durch den Medikamentenabschlussteil an dem ersten Punkt des Medikamentenaufnahmekörperteils zu verlaufen,
wobei der erste Punkt näher an dem Medikamentenabschlussteil angeordnet ist als ein Zentrum des Medikamentenaufnahmekörperteils.

5. Das Verfahren nach Anspruch 4, wobei ein Abstand (E1) vom Medikamentenabschlussteil zum ersten Punkt etwa das 0,7- bis 0,9-fache eines Abstands (E2) vom Medikamentenabschlussteil zur Mitte des Medikamentenaufnahmekörperteils beträgt.

6. Das Verfahren nach Anspruch 4, wobei der Abstand (E1) vom Medikamentenabschlussteil zum ersten Punkt etwa das 0,75- bis etwa 0,85-fache eines Abstands (E2) vom Medikamentenabschlussteil zur Mitte des Medikamentenaufnahmekörperteils beträgt.

7. Das Verfahren nach Anspruch 6, wobei die Dicke (t1) des Medikamentenverbindungsteils größer ist als sowohl die Dicke (t2) des Medikamentenaufnahmekörperteils als auch die Dicke (t3) des Medikamentenabschlussteils.

8. Das Verfahren nach Anspruch 7, wobei die Dicke (t2) des Medikamentenaufnahmekörperteils und die Dicke (t3) des Medikamentenabschlussteils gleich sind, und
die Dicke (t1) des Medikamentenverbindungsteils etwa das 2- bis 4-fache der Dicke (t2) des Medikamentenaufnahmekörperteils beträgt.

9. Das Verfahren nach Anspruch 8, wobei der Körperteil ferner einen Einführstützteil umfasst, der einen ersten Einführstützteil, das von einem distalen Ende des oberen Einführteils nach oben vorsteht, und einen zweiten Einführstützteil umfasst, das von einem distalen Ende des unteren Einführteils nach unten vorsteht.

10. Das Verfahren nach Anspruch 9, wobei das Abdeckteil (3) umfasst:
einen Abdeckkörperteil (31), der in einer Plattenform bereitgestellt ist und so angeordnet ist, dass er von dem Körperstützteil beabstandet ist;
einen Abdeckvorsprung (33), der in einer Form vorsteht, die der des Medikamentenabgabeteils auf einer Oberfläche des Abdeckkörperteils entspricht, die dem Medikamentenabgabeteil zugewandt ist; und
einen Abdeckkörper-Entfernungsteil (35), der sich von dem Abdeckkörperteil zu beiden Enden des Körperstützteils erstreckt und dazu vorgesehen ist, eine Querschnittsfläche aufzuweisen, die von dem Abdeckkörperteil zu dem Körperstützteil allmählich abnimmt,
wobei der Abdeckvorsprung (33) Folgendes umfasst:
einen Abdeckvorsprungskörperteil mit einem Innendurchmesser, der größer ist als ein Außendurchmesser des Medikamentenabgabeteils; und
einen zentralen Teil des Abdeckvorsprungs, der dazu vorgesehen ist, in einer Richtung weg von dem Medikamentenabgabeteil auf einer Oberfläche des Abdeckvorsprungskörperteils, die dem Medikamentenabgabeteil zugewandt ist, vertieft zu sein.

11. Das Verfahren nach Anspruch 10, wobei ein Außendurchmesser (D1) des Abdeckvorsprungskörperteils etwa dem 1,1- bis etwa 1,3-fachen eines Außendurchmessers (D2) des Medikamentenabgabeteils beträgt.

## Revendications

1. Procédé de fabrication d'un tube à médicament de type goutte ;
le tube à médicament de type goutte (T) comprenant :
une partie de corps (1), qui définit une apparence extérieure et dans laquelle un médicament est logé ; et
une partie de couvercle (3), intégrée avec la partie de corps (1) et prévue pour être retirée lorsque le médicament est utilisé,
dans lequel la partie de corps (3) comprend :
une partie de logement de médicament (11), ayant un espace interne (V) pour loger le médicament ;
une partie de support de corps (13), prévue pour s'étendre vers l'extérieur de la partie de logement de médicament (11) ;
une partie d'insertion supérieure (15), s'étendant de manière inclinée vers le haut depuis la partie de logement de médicament (11) et de la partie de support de corps (13), de manière à être éloignée de la partie de couvercle (3) ; et
une partie d'insertion inférieure (17), prévue pour faire face à la partie d'insertion supérieure (15), et s'étendant de manière inclinée vers le bas depuis la partie de logement de médicament (11) et de la partie de support de corps (13), de manière à être éloignée de la partie de couvercle (3),
le procédé (S) comprenant :
un processus de mise en place (S1) pour disposer un premier moule pour former de manière intégrale une apparence extérieure de la partie de couvercle avec la partie de corps, et un deuxième moule pour former l'espace interne de la partie de logement de médicament ;
un processus d'injection (S2) pour entrer un matériau de moulage entre le premier moule et le deuxième moule ;
un processus de retrait (S3) pour retirer le deuxième moule entre la partie d'insertion supérieure et la partie d'insertion inférieure ;
un processus d'entrée (S4) pour entrer le médicament entre la partie d'insertion supérieure et la partie d'insertion inférieure ; et
un processus de liaison (S5) pour lier la partie d'insertion supérieure à la partie d'insertion inférieure
dans lequel le moulage par injection (S2) est effectué dans un état dans lequel un moule est injecté entre la partie d'insertion supérieure (15) et la partie d'insertion inférieure (17) afin de définir l'espace interne (V), et
après le moule est retiré, le médicament est placé dans l'espace interne (V), et la partie d'insertion supérieure (15) et la partie d'insertion inférieure (13) sont liées l'une à l'autre.

2. Le procédé de la revendication 1, dans lequel un angle (θ) entre la partie d'insertion supérieure et la partie d'insertion inférieure est réglé à environ 10 degrés jusqu'à environ 20 degrés.

3. Le procédé de la revendication 2, dans lequel l'angle (θ) entre la partie d'insertion supérieure et la partie d'insertion inférieure est réglé à environ 12,5 degrés jusqu'à environ 17,5 degrés.

4. Le procédé de la revendication 3, dans lequel la partie de logement de médicament (11) comprend :
une partie de décharge de médicament (111), qui est intégrée avec la partie de couvercle et à travers laquelle le médicament est déchargé lorsque la partie de couvercle est retirée ;
une partie de connexion de médicament (113), s'étendant depuis la partie de décharge de médicament, de manière à être éloignée de la partie de couvercle, et ayant un diamètre croissant ;
une partie de corps de logement de médicament (115), s'étendant depuis la partie de décharge de médicament, de manière à être éloignée de la partie de couvercle, et ayant un diamètre constant ; et
une partie de finition de médicament (117), s'étendant depuis la partie de décharge de médicament, de manière à être éloignée de la partie de couvercle, et ayant un diamètre décroissant,
dans lequel la partie d'insertion supérieure (15) est prévue pour passer à travers la partie de finition de médicament à un premier point de la partie de corps de logement de médicament, et
la partie d'insertion inférieure (17) est prévue pour passer à travers la partie de finition de médicament au premier point de la partie de corps de logement de médicament,
dans lequel le premier point est disposé plus près de la partie de finition de médicament qu'un centre de la partie de corps de logement de médicament.

5. Le procédé de la revendication 4, dans lequel une distance (E1) depuis la partie de finition de médicament jusqu'au premier point est environ 0,7 fois à environ 0,9 fois une distance (E2) depuis la partie de finition de médicament jusqu'au centre de la partie de logement de médicament.

6. Le procédé de la revendication 4, dans lequel une distance (E1) depuis la partie de finition de médicament jusqu'au premier point est environ 0,75 fois à environ 0,85 fois une distance (E2) depuis la partie de finition de médicament jusqu'au centre de la partie de logement de médicament.

7. Le procédé de la revendication 6, dans lequel une épaisseur (t1) de la partie de connexion de médicament est supérieure à chaque d'une épaisseur (t2) de la partie de logement de médicament et d'une épaisseur (t3) de la partie de finition de médicament.

8. Le procédé de la revendication 7, dans lequel l'épaisseur (12) de la partie de corps de logement de médicament et l'épaisseur (t3) de la partie de finition de médicament sont les mêmes, et
l'épaisseur (t1) de la partie de connexion de médicament est environ 2 à environ 4 fois l'épaisseur (12) de la partie de corps de logement de médicament.

9. Le procédé de la revendication 8, dans lequel la partie de corps comprend en outre une partie de support d'insertion comprenant une première partie de support d'insertion faisant saillie vers le haut depuis une extrémité distale de la partie d'insertion supérieure, et une deuxième partie de support d'insertion faisant saillie vers le bas depuis une extrémité distale de la partie d'insertion inférieure.

10. Le procédé de la revendication 9, dans lequel la partie de couvercle (3) comprend :
une partie de corps de couvercle (31), prévue sous forme de plaque et disposée de manière à être espacée de la partie de support de corps ;
une saillie de couvercle (33), faisant saillie, sous une forme correspondant à celle de la partie de décharge de médicament, sur une surface de la partie de corps de couvercle, qui fait face à la partie de décharge de médicament ; et
une partie de retrait de couvercle (35), s'étendant depuis la partie de corps de couvercle aux deux extrémités de la partie de support de corps, et prévue pour avoir une zone transversale, qui décroît progressivement depuis la partie de corps de couvercle à la partie de support de corps,
dans laquelle la saillie de couvercle (33) comprend :
une partie de corps de saillie de couvercle, ayant un diamètre intérieur supérieur à un diamètre extérieur de la partie de décharge de médicament ; et
une partie centrale de saillie de couvercle, prévue pour être en retrait, dans une direction éloignée de la partie de décharge de médicament, sur une surface de la partie de corps de saillie de couvercle, qui fait face à la partie de décharge de médicament.

11. Le procédé de la revendication 10, dans lequel un diamètre extérieur (D1) de la partie de corps de saillie de couvercle est environ 1,1 fois à environ 1,3 fois un diamètre extérieur (D2) de la partie de décharge de médicament.
